(19)

Europäisches
Patentamt
European
Patent Office
Office européen
des brevets

(11) **EP 4 036 216 A1**

(12) **EUROPEAN PATENT APPLICATION**
published in accordance with Art. 153(4) EPC

(43) Date of publication:
**03.08.2022 Bulletin 2022/31**

(21) Application number: **20869712.8**

(22) Date of filing: **22.09.2020**

(51) International Patent Classification (IPC):
**C12N 1/12** *(2006.01)*     **C12P 23/00** *(2006.01)*
**C12R 1/89** *(2006.01)*

(52) Cooperative Patent Classification (CPC):
**C12N 1/12; C12N 1/125; C12P 23/00;**
C12R 2001/89

(86) International application number:
**PCT/CN2020/116775**

(87) International publication number:
**WO 2021/057709 (01.04.2021 Gazette 2021/13)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **23.09.2019 CN 201910900994**

(71) Applicants:
- **Bioalgo (WF) Co., Ltd
Weifang, Shandong 261205 (CN)**
- **Bioalgo (CY) Co., Ltd
Changyi Weifang, Shandong 261313 (CN)**

(72) Inventors:
- ZHENG, Yubin
Weifang, Shandong 261205 (CN)
- CAO, Peixin
Weifang, Shandong 261205 (CN)
- LIU, Shuaishuai
Weifang, Shandong 261205 (CN)
- WANG, Hua
Weifang, Shandong 261205 (CN)
- CHEN, Fangjian
Tianjin 300308 (CN)
- XIE, Shiyi
Weifang, Shandong 261205 (CN)

(74) Representative: **Vossius & Partner
Patentanwälte Rechtsanwälte mbB
Siebertstraße 3
81675 München (DE)**

(54) **METHOD FOR CULTURING HAEMATOCOCCUS PLUVIALIS TO PRODUCE ASTAXANTHIN**

(57) A method for producing astaxanthin, comprising: (a) acquiring vegetative cells of astaxanthin-producing *Haematococcus pluvialis;* (b) heterotrophically culturing the vegetative cells of astaxanthin-producing *Haematococcus pluvialis* in a nutrient-poor culture medium containing an organic carbon source and under a no-light condition, to obtain spore cells; and (c) harvesting the spore cells and/or astaxanthin, and optionally purifying the astaxanthin. Also provided is a culture medium used in the described method.

EP 4 036 216 A1

## Description

## Technical Field

The present invention relates to the field of producing astaxanthin, in particular to a method of producing astaxanthin by using *Haematococcus pluvialis,* and to a culture medium used in the method.

## Background Art

Astaxanthin, with a chemical name 3,3'-dihydroxy-4,4'-diketo-β-carotene, a molecular formula of $C_{40}H_{52}O_4$, and a molecular weight of 596.86, is a keto-carotenoid, and has a strong antioxidant function and coloring effect, and therefore is widely used in the fields such as functional foods, cosmetics, feed additives and the like.

Although astaxanthin can be produced via chemical synthesis, its antioxidant activity and biosafety are not as good as natural astaxanthin. *Haematococcus pluvialis* can comprise astaxanthin of up to 1.5-3% of cell dry weight, and with its biosafety has been accepted by major countries in the world, has been approved as a food raw material by the European Union FSA, the US FDA, and the Chinese Ministry of Health and considered as the best organism to produce natural astaxanthin in nature.

*Haematococcus pluvialis* cells are present as motile green vegetative cells in a nutrient-rich environment with appropriate light and temperature, and as thick-walled immotile cells under an unfavorable condition, such as high light intensity, high temperature, high salt, and lack of nutrients, while a large amount of astaxanthin is accumulated to combat against adverse conditions. According to its physiological characteristics, at present, a two-step method is mainly adopted to cultivate *Haematococcus pluvialis* to produce astaxanthin, comprising the first step of expanding green vegetative cells, and the second step of inducing astaxanthin. Cyanotech, a Hawaiian company in the United States, employs an autotrophic method to cultivate *Haematococcus pluvialis* vegetative cells in a closed photobioreactor, and then utilizes sunlight in an open runaway pond to induce the cells to turn red, such that the content of astaxanthin can reach 1.5% of cell dry weight. Algatechologies in Israel uses a pipeline photobioreactor for cultivation of vegetative cells and induction of astaxanthin, with a content of astaxanthin of 3%. The invention patent (PCT/CN2013/084262) discloses a method of producing astaxanthin by using *Haematococcus pluvialis,* comprising obtaining green vegetative cells by means of heterotrophic culture in the first step, adding a culture medium for dilution, and then accumulating astaxanthin via culturing under light, resulting in a content of astaxanthin of 2.3%.

At present, it is generally believed that high light intensity is a key factor for obtaining a high level of astaxanthin. Under a high light condition, algal cells generate excessive reactive oxygen species (ROS) through photosynthesis. *Haematococcus pluvialis* withstands oxidative damage to algae cells due to reactive oxygen species via synthesis and accumulation of astaxanthin (Li et al. 2008, Consump¬tion of oxygen by astaxanthin biosynthesis: a protective mechanism against oxidative stress in Haematococcus pluvialis (Chlorophyceae). J. Plant Physiol. 165:1783- 1797; and Li et al. 2010, Effect of pho¬ton flux densities on regulation of carotenogenesis and cell viability of Haematococcus pluvialis (Chlorophyce¬ae). J. Appl. Phycol. 22:253-263). Therefore, whether green vegetative cells of *Haematococcus pluvialis* are obtained via autotrophic or heterotrophic culture, the induction of astaxanthin must be carried out under light condition, and the higher the light intensity to algal cells, the higher the content of astaxanthin. However, light penetration is inversely related to the density of cells, so reducing the density of cells, increasing specific surface area of reactors, reducing light path of reactors and supplementing artificial light are effective means to increase the content of astaxanthin, but it will lead to problems such as small volume, large quantity, large area, high cost and difficulty in scaling-up of reactors. In addition, in order to reduce production costs, large-scale cultivation of *Haematococcus pluvialis* is performed under sunlight, while duration and intensity of natural light vary with seasons and weather conditions, so the content of astaxanthin is unstable.

It has been reported in literatures that high salinity stimulation can induce the conversion of green vegetative cells of *Haematococcus pluvialis* into red spore cells under a dark condition, but the content of astaxanthin is only 0.5% (30 pg/cell), which has no value of practical application (Kobayashi et al. 1997, Light-independent, astaxanthin production by the green microalga Haematococcus pluvialis under salt stress. Biotechnol Lett 19(6):507-509). Therefore, if a method can be developed to induce *Haematococcus pluvialis* to accumulate a high level of astaxanthin in the absence of light, it will help to change the current state of agricultural production that depends on natural conditions and realize industrialized production.

## Summary of the Invention

Unless otherwise defined, all the technical and scientific terms used herein have the same meanings as commonly understood by a person skilled in the art. For example, refer to Singleton et al., DICTIONARY OF MICROBIOLOGY AND MOLECULAR BIOLOGY 2nd ed., J. Wiley & Sons (New York, NY 1994); Sambrook et al., MOLECULAR CLONING, A LABORATORY MANUAL, Cold Springs Harbor Press (Cold Springs Harbor, NY 1989).

The present invention provides a method of producing astaxanthin by culturing *Haematococcus pluvialis* under a dark condition.

In an embodiment, the present invention provides a method of producing astaxanthin, comprising:

(a) obtaining vegetative cells of astaxanthin-producing *Haematococcus pluvialis;*
(b) heterotrophically culturing the vegetative cells in a nutrient-deficient culture medium containing an organic carbon source under a dark condition to obtain spore cells; and
(c) harvesting the spore cells and/or astaxanthin, optionally purifying astaxanthin.

As used herein, *Haematococcus pluvialis* is a single-cell green alga, belonging to Chlorophyta, Chlorophyceae, Volvocales, Haematococcaceae, and Haematococcus. Under a suitable environment and a nutrient-rich condition, *Haematococcus pluvialis* grows rapidly, divides and reproduces, producing a large number of motile vegetative cells with flagella. When environmental condition becomes unsuitable, the motile cells lose the flagella and become immotile vegetative cells. Under stimulation with a continuous adverse environmental condition, such as high light, high salt, nutrient starvation, etc., vegetative cells no longer divide and reproduce, and fight against the adverse environmental condition by accumulating a large amount of astaxanthin in cells, becoming red chlamydospores.

As used herein, the culture medium for producing astaxanthin can be any culture medium that can be used to cultivate *Haematococcus pluvialis* to allow it grow and reproduce and usually contains a nitrogen source, a phosphorus source, a sulfur source, a magnesium source, a calcium source, and/or a trace element, with amounts that can be determined by a person skilled in the art based on the knowledge and practice in the art. A culture medium suitable for a specific alga is known in the art, c.f. the medium such as BG-11, BBM, C medium, MCM and the like.

The "nitrogen source" that can be used in the culture medium according to the invention refers to an inorganic or organic nitrogen source that can be utilized by cultivated algae, e.g. including but not limited to nitric acid, nitrate, nitrite, aqueous ammonia, ammonium salt, urea, amino acid, peptone, yeast extract, protein powder, corn steep liquor, and any combination thereof.

The "phosphorus source" that can be used in the culture medium according to the invention refers to a phosphorus source that can be utilized by cultivated algae, e.g. including but not limited to phosphoric acid, sodium dihydrogen phosphate, disodium hydrogen phosphate, dipotassium hydrogen phosphate, potassium dihydrogen phosphate, and any combination thereof.

The "sulfur source" that can be used in the culture medium according to the invention refers to a sulfur source that can be utilized by cultivated algae, e.g. including but not limited to sulfuric acid, magnesium sulfate, sodium sulfate, and any combination thereof.

The "magnesium source" that can be used in the culture medium according to the invention refers to a magnesium source that can be utilized by cultivated algae, e.g. including but not limited to magnesium sulfate, magnesium chloride, and any combination thereof.

The "calcium source" that can be used in the culture medium according to the invention refers to a calcium source that can be utilized by cultivated algae, e.g. including but not limited to calcium chloride, calcium sulfate, calcium nitrate, and any combination thereof.

The "trace element" that can be used in the culture medium according to the invention refers to a trace element that can be utilized by cultivated algae, e.g. including but not limited to one or more of Mn (such as manganese chloride), Zn (such as zinc sulfate), B (such as boric acid), I, Mo (such as sodium molybdate), Cu (such as copper sulfate), Co (such as cobalt chloride), Fe (such as ferric chloride). The trace element can be added in the culture medium in an amount determined based on conventional knowledge in the field.

The "organic carbon source" that can be used in the culture medium according to the invention refers to an organic carbon source that can be utilized by target microorganisms to be cultured. Those skilled in the art can determine which organic carbon sources can be used in the culture medium according to the invention based on the technical knowledge in the art, for example, including but not limited to acetic acid, acetate, propionic acid, propionate, butyric acid, butyrate, lactic acid, lactate, fatty acid, fatty acid salt, amino acid, methanol, ethanol, glycerin, citric acid, citrate, pyruvic acid, pyruvate, glucose, fructose, arabinose, lactose, mannose, rhamnose, ribose or waste water, hydrolysate, zymotic fluid containing these organic carbon sources, and any combination thereof. The organic carbon source can be added in an amount determined according to the conventional knowledge in the field and the actual growth condition of algae cells, which are all within technical ability of those skilled in the art.

As used herein, the inoculation density of the astaxanthin-producing *Haematococcus pluvialis* cells can be any density suitable for growth and reproduction of the astaxanthin-producing *Haematococcus pluvialis* cells, and those skilled in the art can determine an appropriate inoculation density based on their technical knowledge and experience in the art. For example, the inoculation density of the astaxanthin-producing *Haematococcus pluvialis* cells according to the invention can be at least $10^4$ cells/ml of culture medium, for example, $1\text{-}20\times10^4$ cells/ml of culture medium, such as 5, 8 or $10 \times 10^4$ cells/ml of culture medium. Alternatively, the inoculation density of the astaxanthin-producing *Haematococcus pluvialis* cells can be at least 0.5-2.0g cells/L of culture medium, for example, at least about 0.5, 0.6, 0.7, 0.8, 0.9, 1.0, 1.1, 1.2, 1.3, 1.4, 1.5, 1.6, 1.7, 1.8, 1.9, 2.0 g cells/L of culture medium.

As used herein, the "nutrient-deficient culture medium" refers to a culture medium lacking a nutrient element, such as one or more or even all of nitrogen source, phosphorus source, sulfur source, magnesium source, calcium source, and trace elements.

In one embodiment, the nutrient-deficient culture medium lacks nitrogen source, phosphorus source, sulfur source, calcium source, magnesium source, and/or a trace element.

In one embodiment, the nutrient-deficient culture medium lacks nitrogen source.

In one embodiment, the nutrient-deficient culture medium lacks nitrogen source and phosphorus source.

In one embodiment, the nutrient-deficient culture medium lacks nitrogen source and a trace element.

In one embodiment, the nutrient-deficient culture medium is a culture medium lacking all the above nutrients. In one embodiment, the culture medium lacking all the nutrients is an acetic acid or acetate solution, such as a sodium acetate solution. In a further embodiment, the culture medium lacking all the nutrients is for example an acetic acid solution at a concentration of 60-1050 g/L, for example about 120, 180, 240, 300, 400, 500, 600, 700, 800, 900, 1000 g/L.

As used herein, the "dark condition" refers to a condition where there is no light or light is insufficient for autotrophic cultivation of the *Haematococcus pluvialis.*

As used herein, the "autotrophic" is a cultivation mode that uses an inorganic carbon source such as carbon dioxide, carbonate or bicarbonate for growth and reproduction through photosynthesis under a light condition.

As used herein, the "mixotrophic" is a cultivation mode that uses an organic carbon source for growth and reproduction under a light condition.

As used herein, the "heterotrophic" is a cultivation mode that uses an organic carbon source for growth and reproduction under a dark condition.

In one embodiment, the vegetative cells in step (a) are obtained by culturing astaxanthin-producing *Haematococcus pluvialis* cells. Various culture methods for growth and reproduction of algae cells are known in the art, such as autotrophic, mixotrophic and heterotrophic culture.

Optionally, after culturing the *Haematococcus pluvialis* cells in step (a), the method may comprise steps of removing the culture medium and/or collecting vegetative cells, and optionally concentrating the vegetative cells. The removal of the culture medium, collecting and/or concentrating the vegetative cells may be performed through any suitable method known in the art, such as precipitation (natural sedimentation or centrifugation) or filtration (using a filter or a membrane).

The culture temperature and pH in step (a) may be any temperature or pH suitable for the growth and reproduction of *Haematococcus pluvialis* cells.

In one embodiment, the culturing in step (a) is performed at a pH value of 6.0-9.0, e.g. 6.0-8.0, 7.0-8.0, 7.0-8.5, 7.5-8.0, 7.5-8.5, 8.0-9.0 or 8.5-9.0, such as about 6.5, 7.0, 7.5, 8.0, 8.5 or 9.0. In one embodiment, the culturing in step (a) is performed in a pH in a range of 7.0-8.0. In one embodiment, the culturing in step (a) is autotrophic, and mixed air containing carbon dioxide (for example, 0.5%-5% (v/v)) may be aerated to control the pH. In one embodiment, the culturing in step (a) is mixotrophic and heterotrophic, and an acid (for example, 0.1-10 mol/L hydrochloric acid, sulfuric acid and acetic acid) may be used to control the pH.

In one embodiment, the culturing in step (a) is performed at a temperature of 15 to 25 °C, preferably 20 to 25°C, e.g. about 15°C, 16 °C, 17°C, 18°C, 19°C, 20°C, 21°C, 22°C, 23°C, 24°C or 25 °C.

In one embodiment, in step (a), a culture broth containing at least about 100,000, 200,000, 300,000, 400,000, 500,000, 600,000, 700,000, 800,000, 900,000, 1,000,000, 1,500,000, 2,000,000, 2,500,000, 3,000,000 or more cells (vegetative cells)/ml is obtained.

In one embodiment, the vegetative cells in step (a) are obtained by autotrophic culture of *Haematococcus pluvialis* cells. *Haematococcus pluvialis* cells can be autotrophically cultured under any conditions suitable for growth and reproduction with light. In one embodiment, the *Haematococcus pluvialis* cells may be inoculated and cultured in a culture medium containing a nitrogen source (for example, a nitrate such as sodium nitrate), and the light intensity may be, for example, but not limited to 10-100, 10-90, 10 -80, 10-70, 20-90, 20-80, 20-70, 30-90, 30-80, 30-70, 40-60 $\mu E/m^2/s$, for example about 20, 30, 40, 50, 60, 70, 80 or 90 $\mu E/m^2/s$. In autotrophic culture, carbon dioxide or a mixed gas containing carbon dioxide may be aerated to provide an inorganic carbon source, such as a mixed air containing 0.5-1.5% (v/v) carbon dioxide; and the aeration volume may be, for example, 0.05-0.5 vvm, such as 0.1- 0.5 or 0.2-0.5 vvm, for example about 0.05, 0.1, 0.15, 0.2, 0.25, 0.3, 0.35, 0.4, 0.45, 0.5 vvm. The pH of the culture broth may be adjusted by adjusting the content of carbon dioxide and the aeration volume.

In one embodiment, the *Haematococcus pluvialis* cells are cultured mixotrophically or heterotrophically in step (a). In one embodiment, the culture medium for mixotrophic or heterotrophic culture in step (a) comprises an organic carbon source containing 80-700, 90-600, 90-500, 90-400, 100-600, 100-500, 100-400, 100-350 or 120-350 mg/L carbon, and a nitrogen source containing 40-800, 40-700, 40-600, 50-800, 50-700, 50-600, 60-800, 60-700, 60-600, 70-800, 70-700, 70-600, 80-600 mg/L nitrogen, preferably with a mass ratio of carbon to nitrogen of 0.1-10:1, 0.2-10:1, 0.1-5:1, 0.2-5:1 or 0.3-4.5:1, e.g. about 0.3:1, 0.5:1, 1:1, 1.4:1, 1.5, 1.8:1, 2:1, 2.4:1, 3:1, 4:1, 4.5:1, 5:1, 6:1, 7:1, 8:1 or 9:1.

In one embodiment, the vegetative cells in step (a) are obtained by mixotrophic culture of *Haematococcus pluvialis* cells. The mixotrophic culture refers to the growth and reproduction of *Haematococcus pluvialis* cells by utilizing an organic carbon source contained in the culture medium under a light condition. In one embodiment, the culture medium used for mixotrophic culture contains an organic carbon source (such as acetic acid or an acetate such as sodium acetate), optionally a nitrogen source (such as nitric acid or a nitrate such as sodium nitrate), preferably with a mass ratio of carbon to nitrogen of 0.1-10:1, 0.2-10:1, 0.1-5:1, 0.2-5:1 or 0.3-4.5:1, e.g. about 0.3:1, 0.5:1, 1:1, 1.4:1, 1.5, 1.8:1, 2:1, 2.4:1, 3:1, 4:1, 4.5:1, 5:1, 6:1, 7:1, 8:1 or 9:1. The light intensity may be, for example, but not limited to, 10-100, 10-90, 10-80, 10-70, 20-90, 20-80, 20-70, 30-90, 30-80, 30-70, 40-60 $\mu$E/m$^2$/s, e.g. about 20, 30, 40, 50, 60, 70, 80 or 90 $\mu$E/m$^2$/s. In an embodiment of mixotrophic culture in step (a), the dissolved oxygen is controlled to be at 1-50%, such as 5-30% or 5-10%, and the dissolved oxygen may be controlled by, for example, adjusting the aeration volume (such as air) and stirring speed. For example, the aeration volume is 0.05-0.5 or 0.05-0.1 vvm, e.g. about 0.05, 0.1, 0.2, 0.3, 0.4, 0.5 vvm, and/or the stirring speed is 50-150 or 50-80 revolutions per minute, e.g. about 50, 60, 70, 80, 90, 100, 110, 120, 130, 140, 150 revolutions per minute.

In one embodiment, in step (a), mixotrophic culture is carried out by fed-batch culture, wherein feed solution is added to the culture broth. In one embodiment, the feed solution contains a culture medium containing an organic carbon source (such as acetic acid or an acetate such as sodium acetate), and optionally a nitrogen source (such as nitric acid or a nitrate such as sodium nitrate). In one embodiment, the feed solution contains an organic carbon source containing 6-420 g/L carbon, and a nitrogen source containing 0.3-120 g/L nitrogen, preferably the mass ratio of carbon to nitrogen is 1-50:1, e.g. about 1:1, 2:1, 3:1, 4:1, 5:1, 10:1, 15:1, 20:1, 25:1, 30:1, 35:1, 40:1, 45:1 or 50:1. In one embodiment, the feed solution is a concentrated culture medium containing an organic carbon source and optionally a nitrogen source, for example, a 5-50X concentrated culture medium.

In one embodiment, the vegetative cells in step (a) are obtained by heterotrophic culture of *Haematococcus pluvialis* cells. The heterotrophic culture refers to a culture mode that *Haematococcus pluvialis* utilizes an organic carbon source in the culture medium for growth and reproduction under a condition with no light or light insufficient for autotrophic culture. In one embodiment, the *Haematococcus pluvialis* is heterotrophically cultured in a culture medium containing an organic carbon source (such as acetic acid or an acetate such as sodium acetate) and optionally a nitrogen source (such as nitric acid or a nitrate such as sodium nitrate), preferably with a mass ratio of carbon to nitrogen of 0.1-10:1, 0.2-10:1, 0.1-5:1, 0.2-5:1 or 0.3-4.5:1, e.g. about 0.3:1, 0.5:1, 1:1, 1.4:1, 1.5:1, 1.8:1, 2:1, 2.4:1, 2.5:1, 3:1, 3.5:1, 4:1, 4.5:1, 5:1, 5.5:1, 6:1, 6.5:1, 7:1, 7.5:1, 8:1, 8.5:1 or 9:1. In an embodiment, during the heterotrophic culture in step (a), the dissolved oxygen is controlled to be at 1-50%, preferably 5-30%, such as about 15%, 20%, 25%, 30%, 35%, 40%, 45% or 50%, and the dissolved oxygen may be controlled by, for example, adjusting the aeration volume (such as air) and stirring speed. For example, the aeration volume is 0.05-0.5 vvm, e.g. about 0.1, 0.2, 0.3, 0.4, 0.5 vvm, and/or the stirring speed is 50-150 revolutions per minute, e.g. about 50, 60, 70, 80, 90, 100, 110, 120, 130, 140, 150 revolutions per minute.

In one embodiment, the heterotrophic culture in step (a) is carried out by fed-batch culture, wherein a feed solution is added to the culture broth. In one embodiment, the feed solution contains a culture medium containing an organic carbon source (such as acetic acid or an acetate such as acetic acid) and optionally a nitrogen source (such as nitric acid or a nitrate such as sodium nitrate). In one embodiment, the feed solution contains an organic carbon source containing 6-420 g/L carbon, and a nitrogen source containing 0.3-120 g/L nitrogen, preferably with a mass ratio of carbon to nitrogen of 1-50:1, 1-40:1, 1-35:1, 5-50:1, 5-40:1 or 5-35:1, e.g. about 1:1, 2:1, 3:1, 4:1, 5:1, 10:1, 15:1, 20:1, 25:1, 30:1, 35:1, 40:1, 45:1 or 50:1. In one embodiment, the feed solution is a concentrated culture medium containing an organic carbon source and optionally a nitrogen source, for example, a 5-50X concentrated culture medium.

In one embodiment, the vegetative cells in step (a) are obtained by mixotrophic or heterotrophic culture of *Haematococcus pluvialis* cells inoculated into a culture medium containing an organic carbon source and a nitrogen source. Preferably, the culture medium comprises an organic carbon source containing 80-700 mg/L carbon, and a nitrogen source containing 40-800 mg/L nitrogen, preferably with a mass ratio of carbon to nitrogen of 0.1-10:1.

In one embodiment, the vegetative cells in step (a) are obtained by mixotrophic or heterotrophic culture of *Haematococcus pluvialis* cells in a manner selected from the group consisting of batch, fed-batch, semi-continuous and continuous culture. When culturing *Haematococcus pluvialis* cells by fed-batch culture, the feed solution preferably contains 15-1050 g/L, more preferably 15-600 or 60-300 g/L of acetic acid or an acetate, a nitrogen source containing 0.3-120 g/L of nitrogen and 1-50 times concentrated medium, preferably with a mass ratio of carbon to nitrogen of about 1-50:1, 1-40:1, 1-35:1, 5-50:1, 5-40:1 or 5-35:1.

In step (b), the *Haematococcus pluvialis* vegetative cells obtained in step (a) are heterotrophically cultured in a nutrient-deficient culture medium containing an organic carbon source under a dark condition, to stimulate the production of astaxanthin by the algae cells. The inoculation density, culture temperature and pH in step (b) may be any density, temperature and pH value suitable for heterotrophic culture of *Haematococcus pluvialis* vegetative cells.

In one embodiment, the inoculation density of *Haematococcus pluvialis* vegetative cells in step (b) may be at least 0.5-2.0 g cells/L of culture medium, e.g. 0.5-1.7 g cells/L of culture medium, e.g. at least about 0.5, 0.6, 0.7, 0.8, 0.9, 1.0, 1.1, 1.2, 1.3, 1.4, 1.5, 1.6, 1.7, 1.8, 1.9, 2.0 g cells/L of culture medium.

In one embodiment, the culturing temperature in step (b) is 15-35 °C, 20-30 °C or 25-30 °C, e.g. about 15 °C, 16 °C, 17 °C, 18 °C, 19 °C, 20 °C, 21 °C, 22 °C, 23 °C, 24 °C, 25 °C, 26 °C, 27 °C, 28 °C, 29 °C, 30 °C, 31 °C, 32 °C, 33 °C or 34 °C.

In one embodiment, the pH value in step (b) is 6.0-11.0, 7.0-10.0, 7.0-9.0 or 7.5-9.0, e.g. about 6.5, 7.0, 7.5, 8.0, 8.5, 9.0, 9.5, 10.0 or 10.5.

In an embodiment, in step (b), the dissolved oxygen is controlled to be at 20-90%, preferably 30-70%. For example, the dissolved oxygen is controlled by adjusting aeration volume and stirring speed, preferably the aeration volume is 0.2-3.0 vvm, e.g. 0.5, 1.0, 1.5, 2.0, 2.5 vvm, and/or preferably the stirring speed is 100-300 revolutions per minute, e.g. 150, 200, 250 revolutions per minute.

In one embodiment, in step (b), in the nutrient-deficient culture medium containing an organic carbon source, the content of carbon element in the organic carbon source is at least about 200 mg/L, e.g. at least about 250, 300, 400, 500, 600, 700, 800, 900, 1000, 1100, 1200, 1300, 1400, 1500, 1600, 1700, 1800, 1900, 2000, 2100, 2200, 2300, 2400, 2500, 3000, 4000, 5000 mg/L. In one embodiment, in step (b), the organic carbon source contained in the nutrient-deficient culture medium is acetic acid or acetate, for example, of 1-15, 1-14, 1-13, 1-12, 1-11, 1-10, 1-9, 1-8, 2-10, 2-9, 2-8, 3-8, 3.5-8, 4-8 g/L, such as about 1, 2, 3, 4, 5, 6, 7, 8, 9, 10, 11, 12, 13, 14 g/L. In one embodiment, the organic carbon source contained in the nutrient-deficient medium in step (b) is sodium acetate, for example, of 1-12, 1-11, 1-10, 1-9, 1-8, 2-10, 2-9, 2-8, 3-8, 3.5-8, 4-8 g/L, such as about 2, 3, 3.5, 4, 4.5, 5, 5.5, 6, 7, 8, 9 or 10 g/L.

In one embodiment, in step (b), the culturing is carried out in feeding manner, wherein the preferred feeding solution is a nutrient-deficient culture medium (for example, lacking nitrogen, phosphorus and/or a trace element, or lack all nutrients) containing acetic acid or acetate (for example, of 30-1050 g/L, particularly 30-600 or 100-600 g/L, such as about 50, 100, 200, 300, 400, 500, 550, 600, 700, 800, 900, 1000 g/L). In one embodiment, the feeding solution is an acetic acid solution, for example, at a concentration of 60-600 g/L, for example about 60, 120, 180, 240, 300, 400, 550, 560, 565, 570 g/L.

In one embodiment, the pH in step (b) is controlled to be in a range of 7.0-8.5, e.g. by adding 0.1-10 mol/L hydrochloric acid, sulfuric acid, or acetic acid.

In one embodiment, the steps (a) and/or (b) may be carried out in a bioreactor. The bioreactor includes various types of photobioreactors, such as a flat plate bioreactor, a column type bioreactor, a hanging bag type bioreactor, a tube type bioreactor, a runway pond and a fermenter.

In one embodiment, when at least 60% (for example, at least 70%, 80%, 90%, 95%, 99%, or even 100%) of the vegetative cells become spore cells and/or the content of astaxanthin no longer increases, the step (b) is stopped.

The harvesting of algae spore cells and/or astaxanthin in step (c) may be carried out by any known method for harvesting algae spore cells (such as sedimentation or centrifugation) and/or destructing cell walls (through a mechanical, chemical or enzymatic manner) and harvesting astaxanthin, optionally separating and/or purifying astaxanthin by any suitable method.

In one embodiment, the present invention provides a method of producing astaxanthin by culturing *Haematococcus pluvialis* under a dark condition, comprising:

(a) culturing *Haematococcus pluvialis* cells in a culture medium, such as by autotrophic, mixotrophic or heterotrophic culture, preferably culture temperature is controlled to be 15-25°C, and pH is controlled to be 6.0-9.0;
optionally harvesting *Haematococcus pluvialis* cells, preferably obtaining concentrated *Haematococcus pluvialis* cells, for example by natural sedimentation, centrifugation or filtration;
(b) heterotrophically culturing the *Haematococcus pluvialis* cells obtained in step (a) under a dark condition in a nutrient-deficient culture medium by adding an organic carbon source, e.g. through heterotrophic culture in a batch, fed-batch, semi-continuous or continuous culture, preferably culture temperature is controlled to be 15-35 °C, and pH is controlled to be 6.0-11.0 and/or the dissolved oxygen is controlled to be 20-90%; and
(c) optionally collecting algae cells and/or harvesting astaxanthin, and optionally purifying astaxanthin.

In one embodiment, the culture medium for the autotrophic culture of *Haematococcus pluvialis* that can be used in step (a) according to the invention comprises or consists of:

potassium dihydrogen phosphate: 0.05-1 g/L,
magnesium sulfate: 50-500 mg/L,
calcium chloride: 5-50 mg/L,
disodium edetate: 0.5-6 mg/L,
boric acid: 0.5-5 mg/L,

ferric chloride: 100-1000 µg/L,
manganese chloride: 10-100 µg/L,
zinc sulfate: 10-100 µg/L,
sodium molybdate: 10-100 µg/L,
cobalt chloride: 5-50 µg/L,
copper sulfate: 10-100 µg/L,
and optionally other nitrogen sources, such as nitric acid or a nitrate such as sodium nitrate, wherein the content of nitrogen is about 40-800 mg/L,
pH 7.0-8.0 (e.g. 7.5).

In one embodiment, the culture medium for mixotrophic and heterotrophic culture of *Haematococcus pluvialis* that can be used in step (a) according to the invention comprises or consists of:

an organic carbon source: 80-700 mg/L (content of carbon element)
a nitrogen source: 40-800 mg/L (content of nitrogen element), wherein the mass ratio of carbon to nitrogen is 0.1-10:1, e.g. 0.2-10:1, 0.1-5:1, 0.2-5:1, preferably 0.3-4.5:1,
potassium dihydrogen phosphate: 0.05-1 g/L,
magnesium sulfate: 50-500 mg/L,
calcium chloride: 5-50 mg/L,
disodium edetate: 0.5-6 mg/L,
boric acid: 0.5-5 mg/L,
ferric chloride: 100-1000 µg/L,
manganese chloride: 10-100 µg/L,
zinc sulfate: 10-100 µg/L,
sodium molybdate: 10-100 µg/L,
cobalt chloride: 5-50 µg/L,
copper sulfate: 10-100 µg/L,
pH 7.0-8.0 (e.g. 7.5).

In one embodiment, the culture medium comprises an organic carbon source of 90-600, 90-500, 90-400, 100-600, 100-500, 100-400, 100-350 or 120-350 mg/L, for example an organic carbon source of about 90 mg/L, 100 mg/L, 110 mg/L, 120 mg/L, 130 mg/L, 140 mg/L, 150mg/L, 200 mg/L, 210 mg/L, 220 mg/L, 230 mg/L, 240 mg/L, 250 mg/L, 260 mg/L, 270 mg/L, 280 mg/L, 290 mg/L, 300 mg/L or 350 mg/L.

In one embodiment, the organic carbon source comprised in the culture medium includes, but not limited to, acetic acid or an acetate such as sodium acetate, glucose, ribose, and any combination thereof.

In one embodiment, the culture medium comprises a nitrogen source of 40-800, 40-700, 40-600, 50-800, 50-700, 50-600, 60-800, 60-700, 60-600, 70-800, 70-700, 70-600, 80-600 mg/L, for example a nitrogen source of about 50 mg/L, 60 mg/L, 70 mg/L, 80 mg/L, 90 mg/L, 100 mg/L, 150 mg/L, 200 mg/L, 250 mg/L, 300 mg/L, 350 mg/L, 400 mg/L, 450 mg/L, 500 mg/L, 550 mg/L or 600 mg/L.

In one embodiment, the nitrogen source comprised in the culture medium includes, but not limited to, nitric acid or a nitrate such as sodium nitrate, ammonium sulfate, urea and any combination thereof.

In one embodiment, the mass ratio of carbon to nitrogen in the culture medium is about 0.2:1; 0.3:1; 0.4:1; 0.5:1; 1:1; 1.5:1; 2:1; 2.5:1; 3:1; 3.5:1; 4:1; 4.5:1; 5:1; 6:1; 7:1; 8:1 or 9:1, for example 1.4:1; 1.8:1; 2.4:1; 4.4:1.

In one embodiment, the culture medium for heterotrophic culture of *Haematococcus pluvialis* that can be used in step (b) according to the invention comprises or consists of:

potassium dihydrogen phosphate: 0.05-1 g/L,
magnesium sulfate: 50-500 mg/L,
calcium chloride: 5-50 mg/L,
disodium edetate: 0.5-6 mg/L,
boric acid: 0.5-5 mg/L,
ferric chloride: 100-1000 µg/L,
manganese chloride: 10-100 µg/L,
zinc sulfate: 10-100 µg/L,
sodium molybdate: 10-100 µg/L,
cobalt chloride: 5-50 µg/L,
copper sulfate: 10-100 µg/L,
pH 7.0-8.0 (for example 7.5).

As used herein, the "nutrient-deficient culture medium" means that the culture medium does not comprise any nutrient element, or comprises a nutrient element lower than an amount necessary for the growth of target microorganisms, resulting in starving of the target microorganisms for the nutrient element. In one embodiment, the "nutrient-deficient culture medium" means that the culture medium does not comprise the nutrient element.

In one embodiment, the nutrient-deficient culture medium described in step (b) of the present invention is the culture medium according to the invention which does not contain respective nutrient elements. As an example, a nitrogen-deficient culture medium may be a culture medium whose composition is described herein, but without a nitrogen-containing compound (disodium edetate). As another example, a phosphorus-deficient culture medium is a culture medium whose composition is described herein, but without a phosphorus-containing compound (potassium dihydrogen phosphate).

In one embodiment, the culture medium according to the invention comprises potassium dihydrogen phosphate of about 0.05 g/L, 0.1 g/L, 0.2 g/L, 0.3 g/L, 0.4 g/L, 0.5 g/L, 0.6 g/L, 0.7 g/L, 0.8 g/L, 0.9 g/L or 1.0 g/L.

In one embodiment, the culture medium according to the invention comprises magnesium sulfate of about 50 mg/L, 100 mg/L, 150 mg/L, 200 mg/L, 250 mg/L, 300 mg/L, 350 mg/L, 400 mg/L, 450mg/L or 500 mg/L.

In one embodiment, the culture medium according to the invention comprises calcium chloride of about 5 mg/L, 10 mg/L, 11 mg/L, 12 mg/L, 13 mg/L, 14 mg/L, 15 mg/L, 20 mg/L, 25 mg/L, 27 mg/L, 30 mg/L, 35 mg/L, 36 mg/L, 40 mg/L, 45 mg/L or 50 mg/L.

In one embodiment, the culture medium according to the invention comprises disodium edetate of 0.5-5.5 mg/L, e.g. about 0.5 mg/L, 1.0 mg/L, 1.5 mg/L, 2.0 mg/L, 2.5 mg/L, 3.0 mg/L, 3.5 mg/L, 4.0 mg/L, 4.5 mg/L, 5.0 mg/L or 5.5 mg/L.

In one embodiment, the culture medium according to the invention comprises boric acid of about 0.5 mg/L, 1.0 mg/L, 1.5 mg/L, 1.6 mg/L, 1.7 mg/L, 1.8 mg/L, 1.9 mg/L, 2.0 mg/L, 2.5 mg/L, 3.0 mg/L, 3.5 mg/L, 4.0 mg/L, 4.5 mg/L 或 5.0 mg/L.

In one embodiment, the culture medium according to the invention comprises ferric chloride of 100-950, 100-900 or 120-900 $\mu$g/L, e.g. about 110 $\mu$g/L, 120 $\mu$g/L, 130 $\mu$g/L, 140 $\mu$g/L, 150 $\mu$g/L, 200 $\mu$g/L, 250 $\mu$g/L, 300 $\mu$g/L, 400 $\mu$g/L, 500 $\mu$g/L, 600 $\mu$g/L, 700 $\mu$g/L, 800 $\mu$g/L or 900 $\mu$g/L.

In one embodiment, the culture medium according to the invention comprises manganese chloride of 15-100 $\mu$g/L, e.g. 15 $\mu$g/L, 20 $\mu$g/L, 25 $\mu$g/L, 30 $\mu$g/L, 35 $\mu$g/L, 40 $\mu$g/L, 50 $\mu$g/L, 60 $\mu$g/L, 70 $\mu$g/L, 72 $\mu$g/L, 80 $\mu$g/L, 90 $\mu$g/L or 100 $\mu$g/L.

In one embodiment, the culture medium according to the invention comprises zinc sulfate of 10-100, 10-90 or 14-90 $\mu$g/L, e.g. about 10 $\mu$g/L, 11 $\mu$g/L, 12 $\mu$g/L, 13 $\mu$g/L, 14 $\mu$g/L, 15 $\mu$g/L, 20 $\mu$g/L, 25 $\mu$g/L, 30 $\mu$g/L, 35 $\mu$g/L, 36 $\mu$g/L, 40 $\mu$g/L, 50 $\mu$g/L, 60 $\mu$g/L, 66 $\mu$g/L, 70 $\mu$g/L, 80 $\mu$g/L, 88 $\mu$g/L, 90 $\mu$g/L or 95 $\mu$g/L.

In one embodiment, the culture medium according to the invention comprises sodium molybdate of about 10 $\mu$g/L, 15 $\mu$g/L, 20 $\mu$g/L, 25 $\mu$g/L, 30 $\mu$g/L, 35 $\mu$g/L, 40 $\mu$g/L, 45 $\mu$g/L, 50 $\mu$g/L, 60 $\mu$g/L, 70 $\mu$g/L, 80 $\mu$g/L, 87 $\mu$g/L, 90 $\mu$g/L or 100 $\mu$g/L.

In one embodiment, the culture medium according to the invention comprises cobalt chloride of about 5 $\mu$g/L, 10 $\mu$g/L, 15 $\mu$g/L, 20 $\mu$g/L, 25 $\mu$g/L, 30 $\mu$g/L, 33 $\mu$g/L, 35 $\mu$g/L, 36 $\mu$g/L, 40 $\mu$g/L, 45 $\mu$g/L or 50 $\mu$g/L.

In one embodiment, the culture medium according to the invention comprises copper sulphate of 20-100 $\mu$g/L, e.g. about 21 $\mu$g/L, 22 $\mu$g/L, 25 $\mu$g/L, 30 $\mu$g/L, 35 $\mu$g/L, 40 $\mu$g/L, 45 $\mu$g/L, 50 $\mu$g/L, 55 $\mu$g/L, 60 $\mu$g/L, 65 $\mu$g/L, 70 $\mu$g/L, 75 $\mu$g/L, 79 $\mu$g/L, 80 $\mu$g/L, 85 $\mu$g/L, 90 $\mu$g/L, 95 $\mu$g/L or 100 $\mu$g/L.

The present invention provides a method of producing astaxanthin by culturing *Haematococcus pluvialis* under a dark condition, comprising steps of:

(I) cultivating vegetative cells: inoculating *Haematococcus pluvialis* seeds into a bioreactor loaded with a culture medium for autotrophic, mixotrophic or heterotrophic culture, preferably at a culture temperature controlled at 15-25 °C, and a pH controlled at 6.0-9.0; and preferably, stopping the cultivation when the algae cells no longer divide and multiply, and green motile cells become green vegetative cells;
(II) preparing cells: removing the culture medium, for example, removing the culture medium by subjecting the algae broth in step (I) to a process such as natural sedimentation, centrifugation, filtration, etc., to obtain concentrated algal cells; and
(III) heterotrophic induction: inoculating the concentrated algae cells of step (II) to a bioreactor loaded with a nutrient-deficient culture medium for heterotrophic culture by adding an organic carbon source, e.g. in a mode such as batch, fed-batch, semi-continuous or continuous culture, wherein the culture temperature is controlled to be 15-35 °C, the pH is controlled to be 6.0-11.0, and the dissolved oxygen is controlled to be 20-90%. Preferably, the culturing is stopped when the algae cells change from green vegetative cells to red spore cells and the content of astaxanthin no longer increases.

In a preferred embodiment, the present invention provides a method of producing astaxanthin by culturing *Haematococcus pluvialis* under a dark condition, comprising:

(a) heterotrophically culturing astaxanthin-producing *Haematococcus pluvialis* in a culture medium containing an organic carbon source and a nitrogen source in a feeding manner, to obtain vegetative cells, which includes one or more, preferably all of the following:

- an organic carbon source selected from acetic acid or an acetate such as sodium acetate;
- a nitrogen source selected from the group consisting of nitric acid, a nitrate such as sodium nitrate, urea, tryptone and a yeast extract;
- the content of carbon in the organic carbon source is 150-300 mg/L, e.g. 175-300 mg/L;
- the content of nitrogen is 100-600 mg/L;
- the mass ratio of carbon to nitrogen in the culture medium is 0.3-3:1, e.g. 0.3-2.5:1;
- the culture temperature is 20-25 °C;
- the dissolved oxygen is controlled to be 15-30%;
- the pH is controlled to be 7.5-8.0; and
- feeding solution contains an organic carbon source and a nitrogen source, wherein the mass ratio of carbon to nitrogen is about 5-35:1, for example 5-33:1;
- the immotile vegetative cells in the obtained vegetative cells account for at least 80%, preferably 100% of the total number of cells;

(b) heterotrophically culturing the vegetative cells obtained in step (a) in a nutrient-deficient culture medium containing an organic carbon source in a fed-batch manner under a dark condition to obtain spore cells, including one or more, preferably all, of the following:

- an organic carbon source selected from acetic acid or an acetate such as sodium acetate, for example of 4.0-5.5 g/L;
- a culture medium lacking (i) a nitrogen source, (ii) a nitrogen source and a phosphorus source, or (iii) all nutrient elements, where
- the culture temperature is 25-30°C, preferably about 30°C;
- the dissolved oxygen is controlled to be 45-70%;
- the pH is controlled to be 7.5-8.0, preferably about 8.0; and
- feeding solution is (i) a culture medium containing an organic carbon source and lacking a nitrogen source and a phosphorus source, or (ii) a culture medium containing an organic carbon source and lacking all nutrients, preferably an acetic acid solution, for example, at a concentration of 60-300g/L or 180-300g/L; and
- when at least 90%, 95% or 100% of the vegetative cells become spore cells, stopping step (b); and

(c) harvesting the spore cells and/or astaxanthin, and optionally purifying astaxanthin.

In a preferred embodiment, the culture medium according to the invention comprises or consists of:

potassium dihydrogen phosphate: 0.1-0.5 g/L,
magnesium sulfate: 150-400 mg/L,
calcium chloride: 12-50 mg/L,
disodium edetate: 1.0-3.5 mg/L,
boric acid: 2-4 mg/L,
ferric chloride: 150-500 $\mu$g/L,
manganese chloride: 25-75 $\mu$g/L, for example 25-72 $\mu$g/L,
zinc sulfate: 20-50 $\mu$g/L,
sodium molybdate: 20-45 $\mu$g/L,
cobalt chloride: 10-35 $\mu$g/L, for example 10-33$\mu$g/L,
copper sulfate: 30-65 $\mu$g/L,
pH 7.5-8.0.

In the present invention, the content of astaxanthin is assayed as follows:

(1) taking 1mL of the induced algae broth, centrifuging at 8000 rpm for 5 minutes, and discarding the supernatant;
(2) adding 1mL of methanol-potassium hydroxide solution (a mixture of 5% potassium hydroxide and 30% methanol) to the algae cell precipitate, water bathing at 70°C for 10 minutes during which vortex-shaking several times, centrifuging at 8000 rpm for 5 minutes, and then removing the supernatant;

(3) adding 45μL of glacial acetic acid and 1mL of DMSO to the algal cell precipitate, then water bathing at 70°C for 10 minutes during which vortex shaking several times, centrifuging at 8000rpm for 5 minutes, and then taking the supernatant in a clean 5mL centrifuge tube; and

(4) repeating the step (3) 2-3 times until the color of the algae turns white, and measuring the absorbance value $A_{492}$ at a wavelength of 492nm.

The content of astaxanthin in *Haematococcus pluvialis* is calculated according to the following formula: (i) the concentration $C_1$ of total astaxanthin extracted from *Haematococcus pluvialis* $(mg/mL) = (A_{492} * 1000)/(A_{1cm}^{1\%} * 100)$, where $A_{1cm}^{1\%} = 2200$; (ii) the extraction yield of total astaxanthin in *Haematococcus pluvialis:* $\omega(\%) = (C_1 * V)/M * 100\%$, where V= volume of DMSO (mL) (dilution times × times of DMSO extraction), M = dry weight of algae cells contained in 1mL of the *Haematococcus pluvialis* broth (mg).

The *Haematococcus pluvialis* as described herein may be any species of *Haematococcus pluvialis,* for example, including but not limited to *Haematococcus pluvialis* CCTCC M2018809, *Haematococcus pluvialis* AC136, AC143, AC587, AC588 (Algobank-Caen Microalgal Culture Collection of University of Caen Basse-Normandie, France), *Haematococcus pluvialis* ATCC 30453, ATCC 30402 (American Type Culture Collection, USA), *Haematococcus pluvialis* CS-321 (Australian National Algae Culture Collection, Australia), *Haematococcus pluvialis* G 1002 (Culture Collection of Algae of Charles University, Czech Republic), *Haematococcus pluvialis* ETTL 1958/3, TAKACOVAL 1983/1, PRIBYL 2005/4, PRIBYL 2008/3 (Culture Collection of Autotrophic Organisms, Czech Republic), *Haematococcus pluvialis* CCCryo 188-04, CCCryo 189-04, CCCryo 190-04 (Culture Collection of Cryophilic Algae, Germany), *Haematococcus pluvialis* SCCAP K-0084 (Scandinavian Culture Collection of Algae and Protozoa at the University of Copenhagen, Denmark), *Haematococcus pluvialis* IPPAS H-239(Culture Collection of Microalgae, Institute of Plant Physiology, Russian Academy of Science, Russia), *Haematococcus pluvialis* NIVA-CHL 9(Norwegian Institute for Water Research Culture Collection of Algae, Norway), *Haematococcus pluvialis* FWAC 7072, FWAC 7039(Canadian Center for the Culture of Microorganisms, Canada), *Haematococcus pluvialis* CPCC 93 (Canadian Phycological Culture Centre of University of Waterloo, Canada), *Haematococcus pluvialis* ACOI 816, ACOI 815, ACOI 276, ACOI 255, ACOI 133, ACOI 51 (Coimbra Culture Collection of Algae, Portugal), *Haematococcus pluvialis* CCAP 34/1D, CCAP 34/1F, CCAP 34/6, CCAP 34/7, CCAP34/8, CCAP 34/12, CCAP 34/13, CCAP 34/14 (Culture Collection of Algae and Protozoa of the Centre for Hydrology and Ecology, UK), *Haematococcus pluvialis* NIES-144, NIES-2263, NIES-2264, NIES-2265 (Microbial Culture Collection of National Institute for Environmental Studies, Japan), *Haematococcus pluvialis* SAG 192.80, SAG 44.96, SAG 34-1a, SAG 34-1b, SAG 34-1c (Culture Collection of Algae at University of Göttingen, Germany), *Haematococcus pluvialis* CCAC 0055, CCAC 0125, CCAC 0129, CCAC 2072B (Culture Collection of Algae at the University of Cologne, Germany), *Haematococcus pluvialis* UTEX 2505, UTEX 16, UTEX B 294 (University of Texas Culture Collection of Algae, USA), *Haematococcus pluvialis* CWU-MACC20 (Herbarium of Kharkov University-MicroAlgae Cultures Collection, Ukraine), *Haematococcus pluvialis* TISTR 8647 (Thailand Institute of Scientific and Technological Research Culture Collection, Thailand), *Haematococcus pluvialis* FACHB-712, FACHB-827, FACHB-797, FACHB-955, FACHB-1164 (Freshwater Culture Algae Collection at Institute of Hydrobiology, The Chinese Academy of Sciences, China) and *Haematococcus pluvialis* CCMP 3127 (Provasoli-Guillard National Centre for Marine Algae and Microbiota, USA).

*Haematococcus pluvialis* AQHP0 was deposited at the China Center for Type Culture Collection (CCTCC) (Wuhan University, Wuhan, China, 430072) under the deposition number of CCTCC M 2018809 on November 21, 2018.

Unless specified in the context, the word "or" intends to include "and".

As used herein, "optional" or "optionally" refers to the occurrence or non-occurrence of the event or situation described following the word, and the description includes the conditions in which the event or situation occurs or does not occur. For example, a step that is "optionally" comprised refers to the presence or absence of the step.

As used herein, the term "about" refers to a range of values that includes a specific value, which can be reasonably understood by those skilled in the art as similar to the specific value. In some embodiments, the term "about" means within the standard error of a measurement generally accepted in the art. For example, in some embodiments, "about" refers to +/- 10% or 5% of the specified value.

As used herein, when a specific value or ratio is given for a feature in the description, it also covers a range defined by any two values or ratios. For example, when the numbers 1, 2, 3, and 4 are listed, ranges 1-2, 1-3, 1-4, 2-3, 2-4 and 3-4 are also covered.

Compared to the prior art, the present invention has the following advantages and effects:

(1) The present invention provides a method of cultivating *Haematococcus pluvialis* to produce astaxanthin, which overcomes the high requirements for light in a conventional scheme, and may achieve the accumulation of a high level of astaxanthin under a completely dark condition. In some embodiments, the content of astaxanthin in *Haematococcus pluvialis* may reach 2.5% or even 3.21% at the end of astaxanthin induction.

(2) Since the present invention no longer relies on light, the reactor design does not need to consider factors such as specific surface area and light path. Large-volume bioreactors such as fermenters may be used to reduce the number of reactors and floor space, thereby reducing production cost.

(3) The present invention gets rid of the dependence of a conventional large-scale cultivation of *Haematococcus pluvialis* on climate, season and geography, which will promote the transformation of traditional agricultural cultivation mode to industrialized large-scale production.

**Brief Description of the Drawings**

FIG. 1 shows the production of astaxanthin by heterotrophically inducing algal cells obtained by autotrophic culture in a batch manner under a dark condition.

FIG. 2 shows the production of astaxanthin by heterotrophically inducing algal cells obtained by mixotrophic in a fed-batch manner under a dark condition.

FIG. 3 shows the production of astaxanthin by heterotrophically inducing algal cells obtained by heterotrophic culture with sodium acetate and sodium nitrate in a fed-batch manner under a dark condition.

FIG. 4 shows the production of astaxanthin by heterotrophically inducing algal cells obtained by heterotrophic culture with sodium acetate and ammonium sulfate in a fed-batch manner under a dark condition.

FIG. 5 shows the production of astaxanthin by heterotrophically inducing algal cells obtained by heterotrophic culture with sodium acetate and urea in a fed-batch manner under a dark condition.

FIG. 6 shows the production of astaxanthin by heterotrophically inducing algal cells obtained by heterotrophic culture with glucose and sodium nitrate in a fed-batch manner under a dark condition.

FIG. 7 shows the production of astaxanthin by heterotrophically inducing algal cells obtained by heterotrophic culture with sodium acetate, ribose and sodium nitrate in a fed-batch manner under a dark condition.

FIG. 8 shows the production of astaxanthin by heterotrophically inducing algal cells obtained by heterotrophic culture with sodium acetate, a yeast extract and peptone in a fed-batch manner under a dark condition.

**Examples**

The techniques and methods of the present invention are generally carried out according to conventional methods well known in the art and described in the references cited in this specification. The invention is further illustrated by the following examples. However, it should be understood that such example are for illustrative purpose, and any example or combination thereof should not be construed as limiting the scope or embodiments of the present invention. The scope of the present invention is defined by the appended claims. In combination with this specification and common knowledge in the art, a person of ordinary skill in the art may clearly understand the scope defined by the claims. Without departing from the spirit and scope of the present invention, those skilled in the art may make any modification or change to the technical solutions of the present invention, and such modifications and changes are also included in the scope of the present invention.

**Example 1**

A basal culture medium has a formula of: 1.0 g/L potassium dihydrogen phosphate, 500 mg/L magnesium sulfate, 36 mg/L calcium chloride, 5 mg/L disodium edetate, 4.5 mg/L boric acid, 900 $\mu$g/L ferric chloride, 100 $\mu$g/L manganese chloride, 88 $\mu$g/L zinc sulfate, 90 $\mu$g/L sodium molybdate, 50 $\mu$g/L cobalt chloride, and 79 $\mu$g/L copper sulfate. Upon preparation, pH was adjusted to 7.5 with a diluted sulfuric acid or sodium hydroxide solution.

*Haematococcus pluvialis* CCTCC M2018809 (deposited at the China Center for Type Culture Collection (CCTCC)) was inoculated in the sterile basal culture medium containing 1.5 g/L sodium nitrate, and placed in a hanging bag film photobioreactor at an initial cell number of 50,000 cells/mL, wherein the light path of the reactor was 6 cm, the volume was 5 L, the loading volume was 70%, and the culture temperature was 22°C. It was subjected to continuous illumination on one side under a white fluorescent lamp for 24 hours, at a light intensity of 60 $\mu$E/m$^2$/s, and mixed air containing 0.5-1.5% (v/v) carbon dioxide was aerated with an aeration volume of 0.2-0.5 vvm and agitated. The pH of the broth was controlled to be 7.5 by adjusting the content of carbon dioxide and the aeration volume. After 240 hours of autotrophic culture, the algae cells no longer divided and reproduced. The number of immotile vegetative cells accounted for 90% of the total cell number, and the total cell number reached 1.1 million cells/mL.

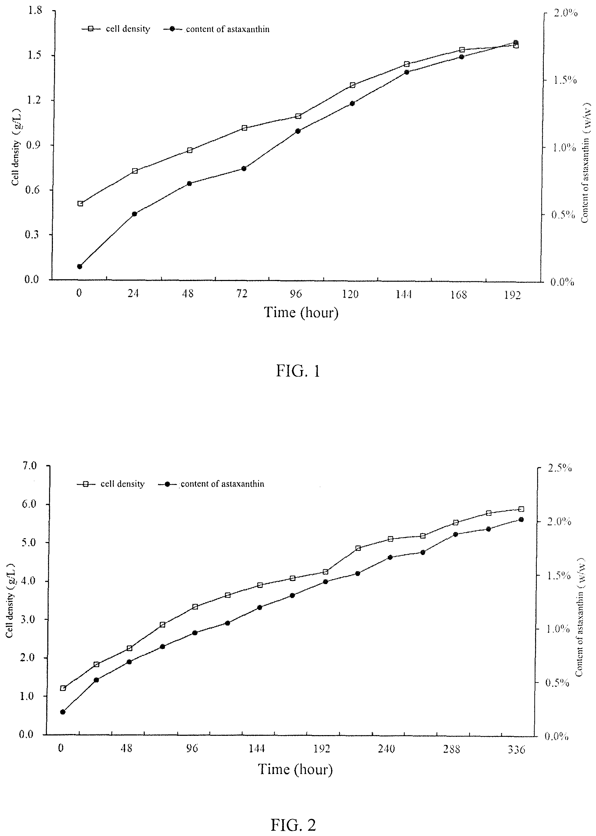

The algae broth was collected and centrifuged in a centrifuge at 3000 rpm for 5 minutes. After removing the supernatant, the concentrated algae cells were inoculated into a nitrogen-deficient basal culture medium containing 8.2 g/L sodium acetate in an inoculation density of 0.51 g/L, and placed in a 0.5L air-lift column reactor, wherein the loading volume was 50%, the air aeration volume was 1.0 vvm, the dissolved oxygen was 30-40% and the culture temperature was 25 °C. The pH was controlled to be 8.5 by adding 0.5 mol/L diluted sulfuric acid. After 192 hours of heterotrophic culture in the absence of light, more than 80% of algae cells changed from green vegetative cells to red spore cells, the algae cell density reached 1.58 g/L, and the content of astaxanthin reached 1.78% (as shown in FIG. 1). The content of astaxanthin was determined through the method as described above.

**Example 2**

A basal culture medium has a formula of: 0.05 g/L potassium dihydrogen phosphate, 50 mg/L magnesium sulfate, 5 mg/L calcium chloride, 0.5 mg/L disodium edetate, 1.9 mg/L boric acid, 120 $\mu$g/L ferric chloride, 15 $\mu$g/L manganese chloride, 14 $\mu$g/L zinc sulfate, 10 $\mu$g/L sodium molybdate, 5 $\mu$g/L cobalt chloride, and 22 $\mu$g/L copper sulfate. Upon preparation, pH was adjusted to 7.0 with a diluted sulfuric acid or sodium hydroxide solution.

*Haematococcus pluvialis* CCTCC M2018809 was inoculated into sterile basal culture medium containing 0.5 g/L sodium acetate (carbon content of 146 mg/L) and 0.5 g/L sodium nitrate (nitrogen content of 82 mg/L), with a ratio of nitrogen to nitrogren of 1.8/1, at an initial number of cells of 80,000 cells/mL, and placed in a 5L glass fermenter with a loading volume of 70% and a culture temperature of 20°C. It was subjected to continuous illumination on one side under a white fluorescent lamp for 24 hours at a light intensity of 40 $\mu$E/m$^2$/s. The dissolved oxygen was controlled to be 5-10% by adjusting air aeration rate at 0.05-0.1 vvm and stirring speed at 50-80 rpm. The pH of the broth was maintained at 7.0 by feeding a 50-times concentrated basal culture medium containing 600 g/L acetic acid and 70 g/L sodium nitrate, and the ratio of carbon to nitrogen in the feeding medium was 20.8/1. After 240 hours of mixotrophic culture, the number of immotile vegetative cells accounted for 85% of the total number of cells, and the total number of cells reached 3 million cells/ml.

Stopping the aeration and stirring, removing the supernatant after natural sedimentation, and inoculating the concentrated algae cells into a nitrogen-deficient basal culture medium containing 6.1 g/L sodium acetate at an inoculation concentration of 1.21 g/L, and placing in a 5L fermenter with a loading volume of 70% and a culture temperature of 25°C. The dissolved oxygen was controlled to be 30-40% by adjusting air aeration volume at 0.2-1.0 vvm and stirring speed at 100-150 rpm. The pH of the broth was maintained at 9.0 by feeding a nitrogen-deficient basal medium containing 565 g/L acetic acid. After 336 hours of heterotrophic culture in the absence of light, more than 70% of algae cells changed from green vegetative cells to red spore cells, the algae cell density reached 5.93 g/L, and the content of astaxanthin reached 2.02% (as shown in FIG. 2). The content of astaxanthin was determined through the method as described above.

**Example 3**

A culture medium has a formula of: 0.5 g/L potassium dihydrogen phosphate, 200 mg/L magnesium sulfate, 12 mg/L calcium chloride, 3 mg/L disodium edetate, 3 mg/L boric acid, 500 $\mu$g/L ferric chloride, 72 $\mu$g/L manganese chloride, 50 $\mu$g/L zinc sulfate, 45 $\mu$g/L sodium molybdate, 33 $\mu$g/L cobalt chloride, and 65 $\mu$g/L copper sulfate. Upon preparation, pH was adjusted to be 8.0 with a diluted sulfuric acid or sodium hydroxide solution.

*Haematococcus pluvialis* CCTCC M2018809 was inoculated into sterile basal culture medium containing 0.8 g/L sodium acetate (carbon content of 234 mg/L) and 0.6 g/L sodium nitrate (nitrogen content of 99 mg/L), with a ratio of carbon to nitrogen of 2.4/1, at an initial number of cells of 100,000 cells/ml, and placed in a 5L fermenter with a loading volume of 70% and a culture temperature of 20°C. The dissolved oxygen was controlled to be 15-20% by adjusting air aeration volume at 0.1-0.4 vvm and stirring speed at 50-100 rpm, and the pH of the broth was maintained at 8.0 by feeding a 5-times concentrated basal culture medium containing 60 g/L acetic acid and 5 g/L sodium nitrate, and the ratio of carbon to nitrogen in the feeding medium was 29.1/1. After 360 hours of heterotrophic culture, algae cells no longer divided and reproduced. The number of immotile vegetative cells accounted for 100% of the total cell number, and the total cell number reached 2.9 million cells/ml.

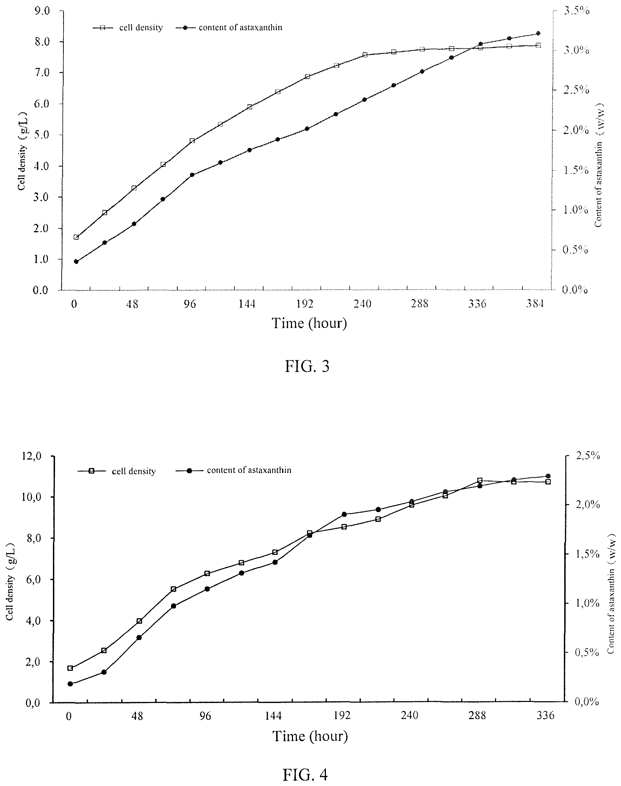

Stopping the aeration and stirring, removing the supernatant after natural sedimentation, abd inoculating the concentrated algae cells into a nitrogen-deficient basal culture medium containing 4.1 g/L sodium acetate at an inoculation concentration of 1.71 g/L, and placing in a 5L fermenter with a loading volume of 70% and a culture temperature of 30°C. The dissolved oxygen was controlled to be 50-70% by adjusting air aeration volume at 1.0-3.0 vvm and stirring speed at 100-200 rpm. The pH of the broth was maintained at 8.0 by feeding a culture medium lacking all nutrients and containing 180 g/L acetic acid. After 384 hours of heterotrophic culture in the absence of light, 100% of algae cells changed from green vegetative cells to red spore cells, the algae cell density reached 7.87 g/L, and the content of astaxanthin reached 3.21% (as shown in FIG. 3). The content of astaxanthin was determined through the method as described above.

**Example 4**

A basal culture medium has a formula of: 0.3 g/L potassium dihydrogen phosphate, 300 mg/L magnesium sulfate, 27 mg/L calcium chloride, 4 mg/L disodium edetate, 3.5 mg/L boric acid, 700 μg/L ferric chloride, 80 μg/L manganese chloride, 90 μg/L zinc sulfate, 87 μg/L sodium molybdate, 40 μg/L cobalt chloride, and 100 μg/L copper sulfate. Upon preparation, pH was adjusted to 7.5 with a diluted sulfuric acid or sodium hydroxide solution.

*Haematococcus pluvialis* CCTCC M2018809 was inoculated into sterile basal culture medium containing 1.2 g/L sodium acetate (carbon content of 351 mg/L) and 0.38 g/L ammonium sulfate (nitrogen content of 81 mg/L), with a ratio of carbon to nitrogen of 4.4/1, at an initial cell number of 100,000 cells/mL and placed in a 5L fermenter with a loading volume of 70% at a culture temperature of 25°C. The dissolved oxygen was controlled to be 10-15% by adjusting air aeration volume at 0.1-0.3 vvm and stirring speed at 50-80 rpm, and the pH of the broth was maintained at 7.5 by feeding a 20-times concentrated basal culture medium containing 180 g/L acetic acid and 11.5 g/L ammonium sulfate, and the ratio of carbon to nitrogen in the feeding medium was 29.5/1. After 312 hours of heterotrophic culture, algae cells no longer divided and reproduced. The number of immotile vegetative cells accounted for 100% of the total cell number, and the total number of cells reached 1 million cells/mL.

Stopping the aeration and stirring, removing the supernatant after natural sedimentation, and inoculating the concentrated algae cells into a basal culture medium absent of all nutrients and containing 6.8 g/L sodium acetate at an inoculation concentration of 1.68 g/L, and placing in a 5L fermenter, with a loading volume of 70% and a culture temperature of 30°C. The dissolved oxygen was controlled to be 35-50% by adjusting air aeration volume at 0.5-1.5 vvm and stirring speed at 100-150 rpm. The pH of the broth was maintained at 8.0 by feeding a nitrogen-deficient basal medium containing 120 g/L acetic acid. After 336 hours of heterotrophic culture in the absence of light, more than 90% of algae cells changed from green vegetative cells to red spore cells, the algae cell density reached 10.7 g/L, and the content of astaxanthin reached 2.29% (as shown in FIG. 4). The content of astaxanthin was determined through the method as described above.

**Example 5**

A basal culture medium has a formula of: 0.2 g/L potassium dihydrogen phosphate, 400 mg/L magnesium sulfate, 50 mg/L calcium chloride, 3.5 mg/L disodium edetate, 4 mg/L boric acid, 200 μg/L ferric chloride, 35 μg/L manganese chloride, 25 μg/L zinc sulfate, 35 μg/L sodium molybdate, 20 μg/L cobalt chloride, and 45 μg/L copper sulfate. Upon preparation, pH was adjusted to 8.0 with a diluted sulfuric acid or sodium hydroxide solution.

*Haematococcus pluvialis* CCTCC M2018809 was inoculated into a sterile basal culture medium containing 1.0 g/L sodium acetate (carbon content of 293 mg/L) and 0.31 g/L urea (nitrogen content of 145 mg/L), with a ratio of carbon to nitrogen of 2.0/1, at an initial cell number of 80,000 cells/mL, and placed in a 5L fermenter with a loading volume of 70%, at a culture temperature of 23°C. The dissolved oxygen was controlled to be 20-25% by adjusting air aeration volume at 0.1-0.4 vvm and stirring speed at 50-100 rpm, and the pH of the broth was maintained at 8.0 by feeding a 10-times concentrated basal culture medium containing 120 g/L acetic acid and 3.1 g/L urea, and the ratio of carbon to nitrogen in the feeding medium was 33.2/1. After 240 hours of heterotrophic culture, algae cells no longer divided and reproduced. The number of immotile vegetative cells accounted for 100% of the total cell number which reached 2.7 million cells/mL.

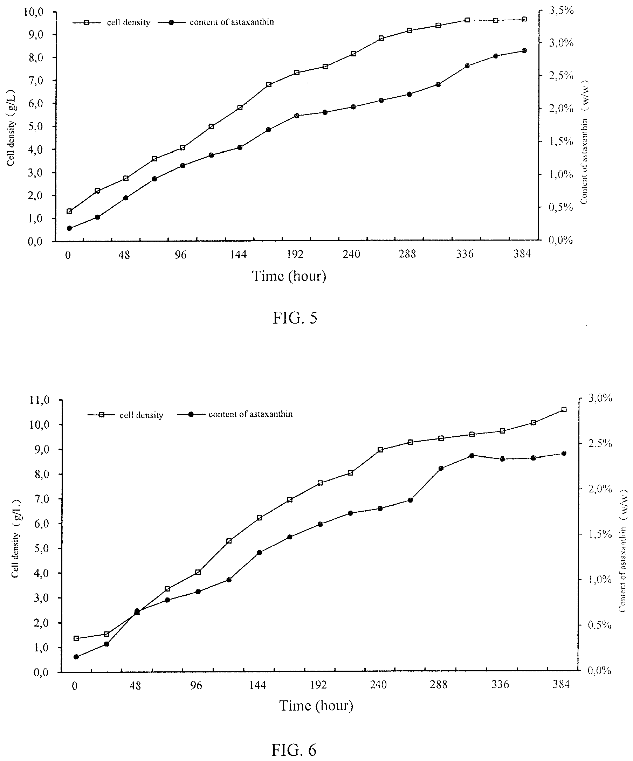

Stopping the aeration and stirring, removing the supernatant after natural sedimentation, and inoculating the concentrated algae cells into a basal culture medium absent of all nutrients and containing 4.5 g/L sodium acetate at an inoculation concentration of 1.31 g/L, and placing in a 5L fermenter, with a loading volume of 70% at a culture temperature of 30°C. The dissolved oxygen was controlled to be 45-60% by adjusting air aeration volume at 1.0-2.0 vvm and stirring speed at 100-200 rpm. The pH of the broth was maintained at 8.0 by feeding a basal culture medium absent of all nutrients and containing 300 g/L acetic acid. After 384 hours of heterotrophic culture in the absence of light, more than 95% of algae cells changed from green vegetative cells to red spore cells, the algae cell density reached 9.60 g/L, and the content of astaxanthin reached 2.88% (as shown in FIG. 5). The content of astaxanthin was determined through the method as described above.

**Example 6**

A basal culture medium has a formula of: 0.6 g/L potassium dihydrogen phosphate, 100 mg/L magnesium sulfate, 10 mg/L calcium chloride, 2 mg/L disodium edetate, 0.5 mg/L boric acid, 600 μg/L ferric chloride, 20 μg/L manganese chloride, 36 μg/L zinc sulfate, 25 μg/L sodium molybdate, 45 μg/L cobalt chloride, and 80 μg/L copper sulfate. Upon preparation, pH was adjusted to 7.5 with a diluted sulfuric acid or sodium hydroxide solution.

*Haematococcus pluvialis* CCTCC M2018809 was inoculated into a sterile basal culture medium containing 0.3 g/L glucose (carbon content of 120 mg/L) and 1.5 g/L sodium nitrate (nitrogen content of 247 mg/L), with a ratio of carbon to nitrogen of 0.5/1 at an initial cell number of 40,000 cells/mL, and placed in a 250 mL Erlenmeyer flask with a loading volume of 100 ml. It was shaking cultured at 100 rpm at a culture temperature of 20°C. After 120 hours of heterotrophic culture, the number of immotile vegetative cells accounted for 70% of the total number of cells, and the total number of cells reached 250,000 cells/mL.

Stopping the aeration and stirring, removing the supernatant after natural sedimentation and inoculating the concentrated algae cells into a basal culture medium lacking nitrogen and phosphorus and containing 3.7 g/L sodium acetate at an inoculation concentration of 1.37 g/L, and placing in a 1L fermenter, with a loading volume of 70% at a culture temperature of 30°C. The dissolved oxygen was controlled to be 45-55% by adjusting air aeration volume at 0.5-1.5 vvm and stirring speed at 100-200 rpm. The pH of the broth was maintained at 7.5 by feeding a basal culture medium absent of all nutrients and containing 400 g/L acetic acid. After 384 hours of heterotrophic culture in the absence of light, more than 85% of algae cells changed from green vegetative cells to red spore cells, the algae cell density reached 10.54 g/L, and the content of astaxanthin reached 2.39% (as shown in FIG. 6). The content of astaxanthin was determined through the method as described above.

**Example 7**

A basal culture medium has a formula of: 0.8 g/L potassium dihydrogen phosphate, 250 mg/L magnesium sulfate, 40 mg/L calcium chloride, 5.5 mg/L disodium edetate, 5 mg/L boric acid, 400 $\mu$g/L ferric chloride, 90 $\mu$g/L manganese chloride, 66 $\mu$g/L zinc sulfate, 100 $\mu$g/L sodium molybdate, 36 $\mu$g/L cobalt chloride, and 90 $\mu$g/L copper sulfate. Upon preparation, pH was adjusted to 7.5 with a diluted sulfuric acid or sodium hydroxide solution.

*Haematococcus pluvialis* CCTCC M2018809 was inoculated into a sterile basal culture medium containing 0.4 g/L sodium acetate (carbon content of 117 mg/L), 0.3 g/L ribose (carbon content of 120 mg/L), and 1.0 g/L sodium nitrate (nitrogen content of 165 mg/L), with a ratio of carbon to nitrogen of 1.4/1 at an initial cell number of 40,000 cells/mL, and placed in a 250 mL Erlenmeyer flask with a loading volume of 100 ml. It was shaking cultured at 100 rpm at a culture temperature of 20°C. After 120 hours of heterotrophic culture, the number of immotile vegetative cells accounted for 60% of the total number of cells, and the total number of cells reached 300,000 cells/mL.

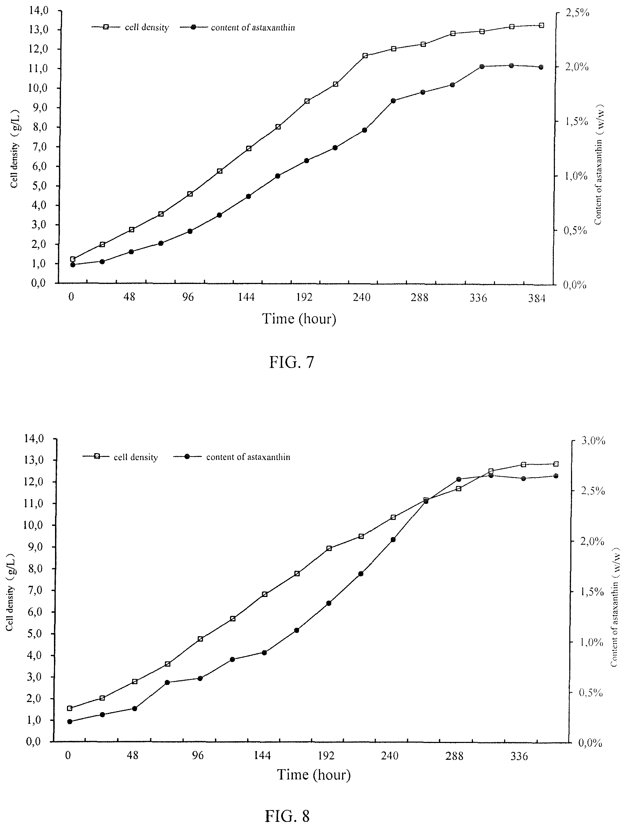

Stopping the aeration and stirring, removing the supernatant after natural sedimentation, and inoculating the concentrated algae cells into a basal culture medium lacking nitrogen and trace elements and containing 5.3 g/L sodium acetate at an inoculation concentration of 1.24 g/L, and placing in a 5L fermenter, with a loading volume of 70% at a culture temperature of 25°C. The dissolved oxygen was controlled to be 30-40% by adjusting air aeration volume at 0.5-1.0 vvm and stirring speed at 100-150 rpm. The pH of the broth was maintained at 7.0 by feeding a basal culture medium lacking nitrogen and trace elements and containing 240 g/L acetic acid. After 384 hours of heterotrophic culture in the absence of light, more than 80% of algae cells changed from green vegetative cells to red spore cells, the algae cell density reached 13.34 g/L, and the content of astaxanthin reached 2.00% (as shown in FIG. 7). The content of astaxanthin was determined through the method as described above.

**Example 8**

A basal culture medium has a formula of: 0.1 g/L potassium dihydrogen phosphate, 150 mg/L magnesium sulfate, 15 mg/L calcium chloride, 1 mg/L disodium edetate, 2 mg/L boric acid, 150 $\mu$g/L ferric chloride, 25 $\mu$g/L manganese chloride, 20 $\mu$g/L zinc sulfate, 20 $\mu$g/L sodium molybdate, 10 $\mu$g/L cobalt chloride, and 30 $\mu$g/L copper sulfate. Upon preparation, pH was adjusted to 7.5 with a diluted sulfuric acid or sodium hydroxide solution.

*Haematococcus pluvialis* CCTCC M2018809 was inoculated into a sterile basal culture medium containing 0.6 g/L sodium acetate (carbon content of 176 mg/L), 4.0 g/L tryptone (nitrogen content of 400 mg/L) and 2.0 g/L yeast extract (nitrogen content of 200 mg/L), with a ratio of carbon to nitrogen of 0.3/1, at an initial cell number of 800,000 cells/mL, and placed in a 5L fermenter with a loading volume of 70% at a culture temperature of 25°C. The dissolved oxygen was controlled to 25-30% by adjusting air aeration volume at 0.2-0.5 vvm and stirring speed at 80-150 rpm, and the pH of the broth was maintained to 7.5 by feeding a 30-times concentrated basal culture medium containing 15 g/L acetic acid, 8.0 g/L tryptone, and 4.0 g/L yeast extract, and the ratio of carbon to nitrogen in the feeding medium was 5.0/1. After 240 hours of heterotrophic culture, the number of immotile vegetative cells accounted for 80% of the total number of cells, and the total number of cells reached 2.8 million cells/mL.

Stopping the aeration and stirring, removing the supernatant after natural sedimentation, and inoculating the concentrated algae cells into a basal culture medium lacking nitrogen and phosphorus and containing 4.9 g/L sodium acetate at an inoculation concentration of 1.55 g/L, and placing in a 5L fermenter, with a loading volume of 70% at a culture temperature of 30°C. The dissolved oxygen was controlled to 50-70% by adjusting air aeration volume at 1.0-2.0 vvm and stirring speed at 100-250 rpm. The pH of the broth was maintained to 8.0 by feeding a basal culture medium lacking nitrogen and phosphorus and containing 60 g/L acetic acid. After 360 hours of heterotrophic culture in the absence of light, more than 90% of algae cells changed from green vegetative cells to red spore cells, the algae cell density reached 12.91 g/L, and the content of astaxanthin reached 2.65% (as shown in FIG. 8). The content of astaxanthin was determined through the method as described above.

**Claims**

1. A method of producing astaxanthin, comprising:

   (a) obtaining vegetative cells of an astaxanthin-producing *Haematococcus pluvialis;*
   (b) heterotrophically culturing the vegetative cells of the astaxanthin-producing *Haematococcus pluvialis* in a nutrient-deficient culture medium containing an organic carbon source under a dark condition to obtain spore cells; and
   (c) harvesting the spore cells and/or astaxanthin, optionally purifying astaxanthin.

2. The method of claim 1, wherein the vegetative cells in step (a) are obtained by autotrophic, mixotrophic or heterotrophic culture of the *Haematococcus pluvialis* cells at a condition:

   (i) culture temperature is controlled to be 15-25°C, such as 20-25°C, and/or
   (ii) the pH is controlled to be 6.0-9.0, preferably 7.0-8.0.

3. The method of claim 1 or 2, wherein the vegetative cells in step (a) are obtained by autotrophic or mixotrophic culture of the *Haematococcus pluvialis* cells, preferably at a light intensity of 10-100 $\mu E/m^2/s$.

4. The method of any one of claims 1-3, wherein the vegetative cells in step (a) are obtained by mixotrophic or heterotrophic culture of the *Haematococcus pluvialis* cells, wherein dissolved oxygen is controlled to be 1-50%, preferably 5-30%.

5. The method of any one of claims 1-4, wherein immotile vegetative cells in the vegetative cells obtained in step (a) account for at least 50%, 60%, 70%, 80%, 85%, 90% or 95%, preferably 100%, of the total number of cells.

6. The method of any one of claims 1 to 5, wherein the vegetative cells in step (a) are obtained by autotrophic culture of the *Haematococcus pluvialis* cells, wherein preferably an inorganic carbon source is provided by aerating a mixed air containing 0.5-1.5% (v/v) carbon dioxide and the pH of the broth is controlled by adjusting the content of carbon dioxide and aeration volume.

7. The method of any one of claims 1 to 6, wherein the vegetative cells in step (a) are obtained by mixotrophic or heterotrophic culture of the *Haematococcus pluvialis* cells in a culture medium containing an organic carbon source and a nitrogen source, wherein preferably the culture medium contains an organic carbon source containing 80-700 mg/L carbon, and a nitrogen source containing 40-800 mg/L nitrogen, preferably the mass ratio of carbon to nitrogen is 0.1-10:1.

8. The method of any one of claims 1 to 7, wherein the vegetative cells in step (a) are obtained by mixotrophic or heterotrophic culture of the *Haematococcus pluvialis* cells in a manner selected from a batch, fed-batch, semi-continuous and continuous culture, wherein, when culturing *Haematococcus pluvialis* cells by a feeding culture, feeding solution preferably contains 15-1050 g/L, more preferably 60-300 g/L of acetic acid or an acetate, a nitrogen source containing 0.3-120 g/L of nitrogen and a 1-50 times concentrated culture medium, preferably the mass ratio of carbon to nitrogen is 1-50:1.

9. The method of claim 7 or 8, wherein the nitrogen source contains an inorganic nitrogen source and/or an organic nitrogen source selected from the group consisting of nitric acid, nitrates, nitrites, aqueous ammonium, ammonium salts, urea, amino acids, peptone, yeast extract, protein powder, corn steep liquor, and any combination thereof.

10. The method of any one of claims 1-9, wherein the organic carbon source is selected from the group consisting of acetic acid, acetates, propionic acid, propionates, butyric acid, butyrates, lactic acid, lactates, fatty acids, fatty acid salts, amino acids, methanol, ethanol, glycerin, citric acid, citrates, pyruvic acid, pyruvates, glucose, fructose, arabinose, lactose, mannose, rhamnose, ribose and waste water, hydrolysate, zymotic fluid containing said organic carbon source(s), and any combination thereof, preferably is acetic acid or an acetate, for example 1-15.0 g/L of acetic acid or an acetate.

11. The method of any one of claims 1-10, wherein the nutrient-deficient culture medium lacks one or more nutrient elements selected from the group consisting of a nitrogen source, a phosphorus source, a sulfur source, a magnesium source, a calcium source and trace elements, more preferably lacks a nitrogen source and/or a phosphorus source and/or a trace element, and more preferably lacks all the nutrient elements, wherein the trace element is one or more selected from the group consisting of Mn, Zn, B, I, Mo, Cu, Co and Fe.

12. The method of any one of claims 1-11, wherein the step (b) comprises one or more of the following:

> (i) the culture medium contains 1-15 g/L acetic acid or an acetate such as sodium acetate,
> (ii) the culture temperature is controlled to be 15-35°C, preferably 25-30°C,
> (iii) the pH is controlled to be 6.0-11.0, preferably 7.0-9.0, and
> (iv) the dissolved oxygen is controlled to be 20 -90%, preferably 30 -70%.

13. The method of any one of claims 1-12, wherein in step (b), heterotrophic culture is carried out in a batch or fed-batch manner, wherein, for fed-batch culture, preferably feeding solution is a nutrient-deficient culture medium containing 15-1050 g/L acetic acid or an acetate or is acetic acid, for example, a nitrogen-deficient culture medium containing 100-600 g/L acetic acid or acetic acid.

14. The method of any one of claims 1-13, wherein, when at least 60% of vegetative cells changed into spore cells and/or the content of astaxanthin no longer increases, the step (b) is stopped.

15. The method of any one of claims 1-14, wherein the culture medium comprises:

| | |
|---|---|
| an organic carbon source | 80-700 mg/L (content of carbon element) |
| a source of nitrogen | 40-800 mg/L (content of nitrogen element) |
| the mass ratio of carbon to nitrogen | 0.1-10:1 |
| potassium dihydrogen phosphate | 0.05-1 g/L, |
| magnesium sulfate | 50-500 mg/L, |
| calcium chloride | 5-50 mg/L, |
| disodium edetate | 0.5-6 mg/L, |
| boric acid | 0.5-5 mg/L, |
| ferric chloride | 100-1000 $\mu$g/L, |
| manganese chloride | 10-100 $\mu$g/L, |
| zinc sulfate | 10-100 $\mu$g/L, |
| sodium molybdate | 10-100 $\mu$g/L, |
| cobalt chloride | 5-50 $\mu$g/L, |
| copper sulfate | 10-100 $\mu$g/L, |
| optionally a pH of 7.0 -8.0. | |

16. The method of any one of claims 1-15, wherein the *Haematococcus pluvialis* is selected from the group consisting of *Haematococcus pluvialis* CCTCC M2018809, AC136, AC143, AC587, AC588, ATCC 30453, ATCC 30402, CS-321, G 1002, ETTL 1958/3, TAKACOVAL 1983/1, PRIBYL 2005/4, PRIBYL 2008/3, CCCryo 188-04, CCCryo 189-04, CCCryo 190-04, SCCAP K-0084, IPPAS H-239, NIVA-CHL 9, FWAC 7072, FWAC 7039, CPCC 93, ACOI 816, ACOI 815, ACOI 276, ACOI 255, ACOI 133, ACOI 51, CCAP 34/1D, CCAP 34/1F, CCAP 34/6, CCAP 34/7, CCAP34/8, CCAP 34/12, CCAP 34/13, CCAP 34/14, NIES-144, NIES-2263, NIES-2264, NIES-2265, SAG 192.80, SAG 44.96, SAG 34-1a, SAG 34-1b, SAG 34-1c, CCAC 0055, CCAC 0125, CCAC 0129, CCAC 2072B, UTEX 2505, UTEX 16, UTEX B 294, CWU-MACC20, TISTR 8647, FACHB-712, FACHB-827, FACHB-797, FACHB-955, FACHB-1164 and CCMP 3127.

17. The method of any one of claims 1-16, comprising:

> (a) heterotrophically culturing an astaxanthin-producing *Haematococcus pluvialis* in a culture medium containing an organic carbon source and a nitrogen source in a fed-batch manner to obtain vegetative cells, comprising one or more, preferably all of the following:

- the organic carbon source is selected from acetic acid or an acetate e.g. sodium acetate;
- the nitrogen source is selected from nitric acid or a nitrate e.g. sodium nitrate, urea, tryptone or a yeast extract;
- the content of carbon element in the organic carbon source is 150-300 mg/L, e.g. 175-300 mg/L;
- the content of nitrogen element is 100-600 mg/L;
- the mass ratio of carbon to nitrogen in the culture medium is 0.3-3:1, e.g. 0.3 -2.5:1;
- culture temperature is 20-25°C;
- dissolved oxygen is controlled to be 15-30%;
- pH is controlled to be 7.5-8.0;
- feeding solution contains an organic carbon source and a nitrogen source, wherein the mass ratio of carbon to nitrogen is 5-35:1, e.g. 5-33:1;
- immotile vegetative cells in the obtained vegetative cells account for at least 80%, preferably 100% of the total number of cells;

(b) heterotrophically culturing the vegetative cells obtained in step (a) in a nutrient-deficient culture medium containing an organic carbon source in a fed-batch manner under a dark condition to obtain spore cells, comprising one or more, preferably all, of the following:

- the organic carbon source is selected from acetic acid or an acetate e.g. sodium acetate, e.g. of 4.0-5.5 g/L,
- the culture medium lacks (i) a nitrogen source, (ii) a nitrogen source and a phosphorus source, or (iii) all nutrient elements,
- culture temperature is 25-30°C, preferably about 30°C;
- dissolved oxygen is controlled to be 45-70%;
- pH is controlled to be 7.5-8.0, preferably about 8.0; and
- feeding solution is (i) a culture medium containing an organic carbon source and lacking a nitrogen source and a phosphorus source or (ii) a culture medium containing an organic carbon source and lacking all nutrients, preferably an acetic acid solution, e.g. at a concentration of 60-300g/L or 180-300g/L; and
- when at least 90%, 95% or 100% of the vegetative cells change to spore cells, stopping the step (b); and

(c) harvesting the spore cells and/or astaxanthin, optionally purifying astaxanthin.

**18.** A culture medium, comprising:

| | |
|---|---|
| an organic carbon source | 80-700 mg/L (content of carbon element) |
| a nitrogen source | 40-800 mg/L (content of nitrogen element) |
| the mass ratio of carbon to nitrogen | 0.1-10:1 |
| potassium dihydrogen phosphate | 0.05-1 g/L, |
| magnesium sulfate | 50-500 mg/L, |
| calcium chloride | 5-50 mg/L, |
| disodium edetate | 0.5-6 mg/L, |
| boric acid | 0.5-5 mg/L, |
| ferric chloride | 100-1000 $\mu$g/L, |
| manganese chloride | 10-100 $\mu$g/L, |
| zinc sulfate | 10-100 $\mu$g/L, |
| sodium molybdate | 10-100 $\mu$g/L, |
| cobalt chloride | 5-50 $\mu$g/L, |
| copper sulfate | 10-100 $\mu$g/L, |
| optionally pH 7.0-8.0. | |

FIG. 1

FIG. 2

FIG. 3

FIG. 4

FIG. 5

FIG. 6

FIG. 7

FIG. 8

## INTERNATIONAL SEARCH REPORT

| International application No. |
| --- |
| **PCT/CN2020/116775** |

**A. CLASSIFICATION OF SUBJECT MATTER**

C12N 1/12(2006.01)i; C12P 23/00(2006.01)i; C12R 1/89(2006.01)n

According to International Patent Classification (IPC) or to both national classification and IPC

**B. FIELDS SEARCHED**

Minimum documentation searched (classification system followed by classification symbols)

    C12N,C12P,C12R

Documentation searched other than minimum documentation to the extent that such documents are included in the fields searched

Electronic data base consulted during the international search (name of data base and, where practicable, search terms used)

DWPI, SIPOABS, CNABS, USTXT, JPTXT, EPTXT, WOTXT, CNTXT, CNKI, ISI WEB OF SCIENCE, PUBMED: 异养, 无光照, 无光, 营养缺失, 虾青素, 孢子细胞, 营养细胞, 磷酸二氢钾, 硫酸镁, 氯化钙, 乙二胺四乙酸二钠, 硼酸, 氯化铁, 氯化锰, 硫酸锌, 钼酸钠, 氯化钴, 硫酸铜, heterotrophic, heterotrophy, without, independent, light, dark, astaxanthin, light-independent, dark, sporoblast, nutritive cell, nutrient deficiency, potassium dihydrogen phosphate, magnesium sulfate, calcium chloride, disodium ethylenediamine tetraacetate, boric acid, ferric chloride, manganese chloride, zinc sulfate, sodium molybdate, cobalt chloride, copper sulphate

**C. DOCUMENTS CONSIDERED TO BE RELEVANT**

| Category* | Citation of document, with indication, where appropriate, of the relevant passages | Relevant to claim No. |
| --- | --- | --- |
| A | CN 107365826 A (SHENZHEN DEHE BIOTECH CO., LTD.) 21 November 2017 (2017-11-21) see claims 1-8 | 1-17 |
| A | US 7063957 B2 (THE UNIVERSITY OF HONG KONG) 20 June 2006 (2006-06-20) see entire document | 1-18 |
| A | CN 106399108 A (SHANDONG JINJING BIOTECHNOLOGY CO., LTD.) 15 February 2017 (2017-02-15) see entire document | 1-18 |
| A | CN 106566775 A (QINGDAO KEHAI BIOLOGICAL CO., LTD.) 19 April 2017 (2017-04-19) see entire document | 1-18 |
| Y | CN 103571906 A (SHANGHAI ZEYUAN MARINE BIOTECHNOLOGY CO., LTD.) 12 February 2014 (2014-02-12) see claims 1-12 | 18 |

☑ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **14 December 2020** | **20 January 2021** |

| Name and mailing address of the ISA/CN | Authorized officer |
| --- | --- |
| **China National Intellectual Property Administration (ISA/CN)** **No. 6, Xitucheng Road, Jimenqiao, Haidian District, Beijing 100088** **China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2020/116775**

### C.     DOCUMENTS CONSIDERED TO BE RELEVANT

| Category* | Citation of document, with indication, where appropriate, of the relevant passages | Relevant to claim No. |
|---|---|---|
| A | Ip, PF et al. "Production of astaxanthin by the green microalga Chlorella zofingiensis in the dark" *PROCESS BIOCHEMISTRY*, Vol. 40, No. 2, 31 December 2005 (2005-12-31), ISSN: 1359-5113, pp. 733-738, see entire document | 1-18 |
| Y | CN 107365826 A (SHENZHEN DEHE BIOTECH CO., LTD.) 21 November 2017 (2017-11-21) see claims 1-8 | 18 |
| A | CN 103571906 A (SHANGHAI ZEYUAN MARINE BIOTECHNOLOGY CO., LTD.) 12 February 2014 (2014-02-12) see entire document | 1-17 |

Form PCT/ISA/210 (second sheet) (January 2015)

INTERNATIONAL SEARCH REPORT
Information on patent family members

International application No.

**PCT/CN2020/116775**

| Patent document cited in search report | | | Publication date (day/month/year) | Patent family member(s) | | | Publication date (day/month/year) |
|---|---|---|---|---|---|---|---|
| CN | 107365826 | A | 21 November 2017 | None | | | |
| US | 7063957 | B2 | 20 June 2006 | US | 2005214897 | A1 | 29 September 2005 |
| CN | 106399108 | A | 15 February 2017 | CN | 106399108 | B | 07 January 2020 |
| CN | 106566775 | A | 19 April 2017 | CN | 106566775 | B | 07 April 2020 |
| CN | 103571906 | A | 12 February 2014 | AU | 2013295436 | A1 | 19 March 2015 |
| | | | | EP | 2878676 | A4 | 25 May 2016 |
| | | | | CN | 103571906 | B | 11 December 2018 |
| | | | | BR | 112015001637 | A2 | 04 July 2017 |
| | | | | EP | 2878676 | A2 | 03 June 2015 |
| | | | | WO | 2014015841 | A3 | 20 March 2014 |
| | | | | US | 2015252391 | A1 | 10 September 2015 |
| | | | | WO | 2014015841 | A2 | 30 January 2014 |
| | | | | IN | 1606DEN2015 | A | 03 July 2015 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


**Patent documents cited in the description**

- CN 2013084262 W

- CN 430072


**Non-patent literature cited in the description**

- **LI et al.** Consump¬tion of oxygen by astaxanthin biosynthesis: a protective mechanism against oxidative stress in Haematococcus pluvialis (Chlorophyceae). *J. Plant Physiol,* 2008, vol. 165, 1783-1797
- **LI et al.** Effect of pho¬ton flux densities on regulation of carotenogenesis and cell viability of Haematococcus pluvialis (Chlorophyce¬ae). *J. Appl. Phycol,* 2010, vol. 22, 253-263

- **KOBAYASHI et al.** Light-independent, astaxanthin production by the green microalga Haematococcus pluvialis under salt stress. *Biotechnol Lett,* 1997, vol. 19 (6), 507-509
- **SINGLETON et al.** DICTIONARY OF MICROBIOLOGY AND MOLECULAR BIOLOGY. J. Wiley & Sons, 1994
- **SAMBROOK et al.** MOLECULAR CLONING, A LABORATORY MANUAL. Cold Springs Harbor Press, 1989